# EUROPEAN PATENT SPECIFICATION

(11) **EP 4 574 186 B1**
(45) Date of publication and mention of the grant of the patent: **12.08.2026**
(21) Application number: 24218306.9
(22) Date of filing: 09.12.2024
(51) Int. Cl.: A61M 5/14, A61M 5/142

(54) **INFUSION MODULE ADAPTER**
INFUSIONSMODULADAPTER
ADAPTATEUR DE MODULE DE PERFUSION

(30) Priority: 20.12.2023 US 202363612541 P
(43) Date of publication of application: 25.06.2025
(73) Proprietor: CareFusion 303, Inc., San Diego, CA 92130 (US)
(72) Inventor: Rozell, Gene A., San Diego, 92130 (US); Burgess, Brendan John, San Diego, 92130 (US)
(74) Representative: Zenz Patentanwälte Partnerschaft mbB

(56) References cited:
- WO-A1-03/006091
- US-A1- 2007 088 249
- US-A1- 2013 317 837
- US-A1- 2020 054 823

## Description

### BACKGROUND

Infusion pumps are complex electromechanical devices used to deliver fluids into a patient's body in a controlled manner. They typically serve the needs of hospital-bound patients, where life-saving medication is normally delivered via intravenous infusion. Infusion pump systems typically comprise a plurality of modules configured for different programming and pumping functions. These modules normally work together, using compatible power specifications, communication protocols, and mechanical interconnects.

US 2020/054823 A1 discloses a pump for treating a patient, the pump includes a spring-biased plunger biased toward actuation against a tube; a cam shaft configured to actuate the spring-based plunger; a lever actuatable between a closed position and an open position; a shaft coupled to the lever, the shaft having a central axis centrally along the length of the shaft, the shaft coupled to the lever to rotate around the central axis in accordance with actuation of the lever; and a lift cam pivotally coupled to the shaft, wherein the lift cam pivots around a lift cam axis, the lift cam axis of the lift cam is parallel to the central axis of the shaft, and the lift cam engages with the spring-based plunger to lift the spring-biased plunger off of the cam shaft as the shaft rotates in accordance with actuating the lever to the open position.

US 2007/088249 Al discloses a modular patient care system having a central management unit module and one or more detachable functional units is described. Using unique mechanical and electrical features, the modular patient care system is capable of flexibly, bilaterally, and safely providing electrical power from the central management unit to the attached functional units, with exposed power leads of end units being electrically isolated for safety and security. Functional units are capable of detecting the presence of other functional units more distant from the central management unit for passing power to those units, and for otherwise electrically isolating exposed power leads when no further units are attached. Additionally, the modular patient care system provides for a modular connection arrangement wherein modules are detachably connected to each other in a convenient, flexible, interchangeable, and secure manner by providing a hinge connector pair, a specially located latch mechanism, and a guide means between any pair of modules.

To maintain cross-compatibility between modules in conventional infusion pump systems, one module typically cannot be upgraded without upgrading the entire system. However, upgrading a fleet of infusion pump systems in a healthcare facility can be cost-prohibitive. Rather than upgrading individual modules or components when desired, it can be more economical to wait until more components of the system are in need of an upgrade before replacing the entire system. However, waiting comes at a cost of delayed access to improvements in efficiency and safety features that come with newer modules. Thus, there is a tradeoff between cost-optimization and feature-optimization when deciding whether and how often to upgrade conventional infusion pump systems.

### SUMMARY

The invention is defined in the independent claims. Preferable embodiments are further laid out in the dependent claims. Based on the discussion above as well as other problems and disadvantages of the related art, there is a need for an infusion pump system that can be upgraded in parts while maintaining cross-compatibility between the various modules included in the system. Such a system solves the trade-off between cost-optimization and feature-optimization by providing the ability to upgrade individual modules (e.g., the main point of care unit) while maintaining functionality of other modules (e.g., pump modules) without requiring every module to be upgraded at the same time. An infusion pump system as described herein uses an infusion module adapter system that joins two or more subsystems having components that are not compatible with each other, thereby providing modularity, physical interoperation, and logical interoperation between the otherwise incompatible subsystems of the infusion pump system.

According to various aspects of the subject technology and according to the invention, an infusion module adapter system couples a control unit of a patient care system to an infusion pump. The adapter system includes a first interconnect system on a first side and a second interconnect system on a second side. The first interconnect system includes a mechanical connector having first support contacts for mechanical coupling to the control unit; and a logical connector having first power contacts and first communication contacts for logical coupling to the control unit. The second interconnect system includes an integrated connector having second support contacts, second power contacts, and second communication contacts for mechanical coupling and logical coupling to the infusion pump. The adapter system further includes power conversion circuitry configured to convert a power signal between the first power contacts and the second power contacts, and processing circuitry configured to convert messages between the first communication contacts and the second communication contacts.

In some implementations, the first support contacts of the mechanical connector of the first interconnect system comprise a latching mechanism that supports physical continuity of attachment to the control unit; and the second support contacts of the integrated connector of the second interconnect system comprise a latching mechanism that supports physical continuity of attachment to the infusion pump.

According to the invention, the first power contacts of the logical connector of the first interconnect system are configured to receive the first power signal from the control unit, wherein the first power signal is characterized by a first voltage, a first amplitude, or a first frequency; the power management module is configured to convert the first voltage, the first amplitude, or the first frequency to a second voltage, a second amplitude, or a second frequency; and the second power contacts of the integrated connector of the second interconnect system are configured to transmit the second power signal to the infusion pump, wherein the second power signal is characterized by the second voltage, the second amplitude, or the second frequency.

In some implementations, the first communication contacts of the logical connector of the first interconnect system are configured to send messages to and receive messages from the control unit; the second communication contacts of the integrated connector of the second interconnect system are configured to send messages to and receive messages from the infusion pump; and the processing circuitry of the communication conversion module is configured to: translate the messages received from the control unit into a message format that is consumable by the infusion pump and send translated control unit messages to the infusion pump; and translate the messages received from the infusion pump into a message format that is consumable by the control unit and send translated infusion pump messages to the control unit.

In some implementations, the messages received from the control unit include pumping instructions. In some implementations, the messages received from the infusion pump include pumping status or alarm data.

In some implementations, the processing circuitry of the communication conversion module is configured to manage state transitions for pump states of the infusion pump. In some implementations, the processing circuitry of the communication conversion module is configured to receive programming updates and facilitate inter-operation for a plurality of infusion pumps.

In some implementations, the infusion module adapter system further includes a wireless transceiver. In some implementations, the processing circuitry of the communication conversion module is configured to receive firmware updates via the wireless transceiver. In some implementations, the processing circuitry of the communication conversion module is configured to transmit logging and/or asset tracking messages via the wireless transceiver.

In some implementations, the processing circuitry of the communication conversion module is configured to verify compatibility with the infusion pump upon the infusion pump being attached to the infusion module adapter system. In some implementations, the processing circuitry of the communication conversion module is configured to verify compatibility by validating an authorization code stored in a data storage of the infusion module adapter system.

In some implementations, the processing circuitry of the communication conversion module is configured to verify compatibility by validating a firmware or software version of (i) the control unit and the infusion module adapter system, (ii) the infusion module adapter system and the infusion pump, and/or (iii) the control unit and the infusion pump.

In some implementations, the processing circuitry of the communication conversion module is configured to verify compatibility by validating cross-compatibility, product-level compatibility, and/or version-level compatibility of (i) the control unit and the infusion module adapter system, (ii) the infusion module adapter system and the infusion pump, and/or (iii) the control unit and the infusion pump.

In some implementations, the mechanical connector of the first interconnect system is a mechanical modular interconnect (M-MIC) connector. In some implementations, the logical connector of the first interconnect system is an electrical modular interconnect (E-MIC) connector. In some implementations, the integrated connector of the second interconnect system is an inter-unit interface (IUI) connector.

In some implementations, the mechanical connector of the first interconnect system and the integrated connector of the second interconnect system are disposed in an upper portion of the infusion module adapter system; and the logical connector of the first interconnect system is disposed in a lower portion of the infusion module adapter system.

In some implementations, the mechanical connector of the first interconnect system is disposed in an upper portion of the infusion module adapter system; and the logical connector of the first interconnect system and the integrated connector of the second interconnect system are disposed in a lower portion of the infusion module adapter system.

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

For a better understanding of the various described implementations, reference should be made to the Detailed Description below, in conjunction with the following drawings. Like reference numerals refer to corresponding parts throughout the drawings and description.
FIG. 1 depicts an example infusion pump system in its intended environment in accordance with some implementations.
FIG. 2 depicts an infusion pump system having a plurality of subsystems that are joinable using compatible mechanical and electrical interconnects.
FIG. 3 depicts an infusion pump system having a plurality of subsystems that are joinable using compatible mechanical and electrical interconnects.
FIG. 4 depicts an infusion pump system having a combination of the subsystems described in FIGS. 3-4, wherein the subsystems are not joinable due to having incompatible mechanical and electrical interconnects.
FIG. 5 depicts an infusion pump system having a plurality of subsystems having incompatible mechanical and electrical interconnects, and a plurality of infusion module adapters having sets of interconnects that are compatible with the different subsystems of the infusion pump system.
FIGS. 6A-6B depict closeup views of different configurations of an infusion module adapter in accordance with some implementations.
FIG. 7 is a conceptual diagram illustrating an example electronic system for joining subsystems having incompatible interconnects when operating an infusion pump.

### DETAILED DESCRIPTION

Reference will now be made to implementations, examples of which are illustrated in the accompanying drawings. In the following description, numerous specific details are set forth, in order to provide an understanding of the various described implementations. However, it will be apparent to one of ordinary skill in the art that the various described implementations may be practiced without these specific details. In other instances, well-known methods, procedures, components, circuits, and networks have not been described in detail so as not to unnecessarily obscure aspects of the implementations.

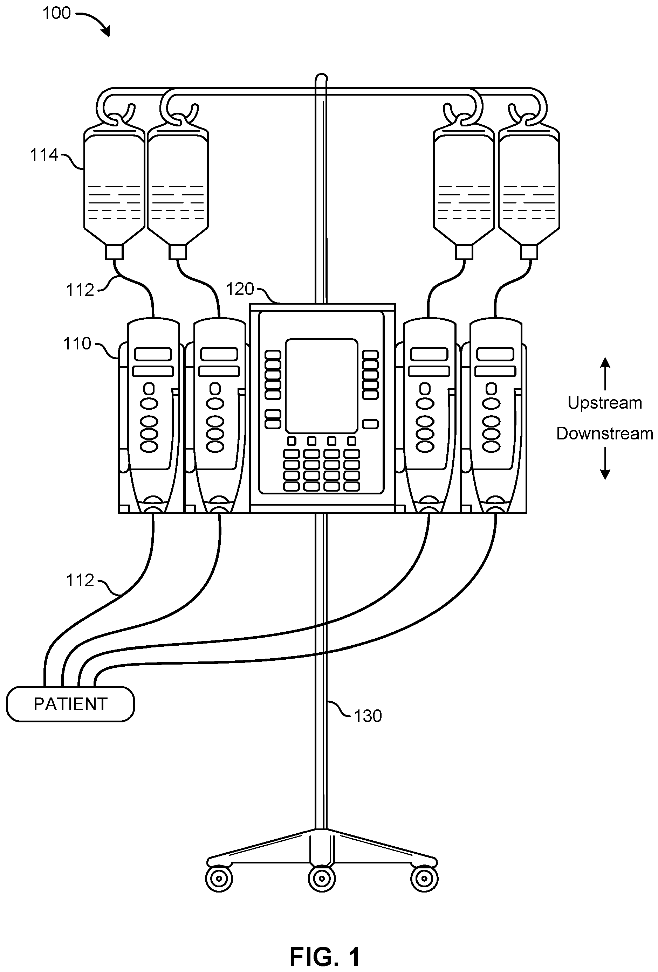

FIG. 1 depicts an example patient care system 100, in accordance with some implementations. The patient care system 100 includes a plurality of infusion pumps 110 (e.g., large-volume pumps (LVPs)) and a control unit 120 (sometimes referred to as an interface unit). The infusion pumps 110 are used to accurately deliver liquids through intravenous or epidural routes to a patient for therapeutic and/or diagnostic purposes, normally with rates from 0.1 to 999 mL/hr or higher. The control unit 120 (also referred to as an infusion controller) is an essential programming unit that drives communications and interactions prior to and during an infusion procedure. The control unit 120 may include a display and an interface (e.g., keypad or touchscreen), which trained care professionals use to program the infusion pumps 110. Each infusion pump 110 may be programmed with a specific amount of fluid to deliver into the patient, one or more timing parameters for delivering the fluid, and other specific instructions.

In the depicted example, the patient care system 100 includes four fluid infusion pumps 110, each of which is in operative engagement with a respective fluid administration set 112. The infusion pumps 110 are directly or indirectly connected to and controlled by a control unit 120. Fluid supplies 114, which may take various forms but in this case are shown as bags, are inverted and suspended above respective infusion pumps 110. Fluid supplies 114 may also take the form of bottles or other types of containers. The control unit 120 and the fluid supplies 114 are mounted to a roller stand or pole 130. Each fluid supply 114 is connected to a patient by a respective administration set 112 so that the patient may receive the fluids in all of the fluid supplies 114.

In the depicted example, a separate infusion pump 110 is used to infuse each of the fluids of the fluid supplies 114 into the patient. The infusion pumps 110 are flow control devices that act on the respective tube or fluid conduit of a corresponding administration set 112 to move the fluid from the fluid supply 114 through the conduit and to the patient. Because individual infusion pumps 110 are used, each pump may be individually set to the infusion pumping or operating parameters required for infusing the particular medical fluid from the respective fluid supply 114 into the patient at a particular rate prescribed for that fluid by a clinician.

In some implementations, each infusion pump 110 may include various sensors and detectors that are configured to monitor the fluid flow and detect hazards. For example, air-in-line (AIL) detectors may be configured to detect air in the tubing of the administration sets 112. In another example, occlusion sensors may be configured to detect pressure changes in the upstream or downstream tubing of the administration sets 112, indicating presence of an occlusion in the line. Various other hazard sensors may be configured to monitor any other aspect of the infusion process associated with a respective infusion pump 110. The aforementioned sensors may monitor air, pressure, flow, or any other infusion metric at intervals or for a time series by sampling values over time. When these metrics are collected for analysis, the collected metrics may reflect an average, a moving average, a maximum for period, a minimum for period, correspondence to a threshold or range, or the like, of the underlying values detected by corresponding sensors or detectors.

In some implementations, any or all of the aforementioned sensors and detectors may be located inside respective infusion pumps 110. In some implementations, any or all of the aforementioned sensors and detectors may be located upstream or downstream of the infusion pumps 110, as long as a portion of each administration set 112 passes through or in proximity to a respective sensor or detector.

The infusion pumps 110 may be configured to adjust their operation in accordance with the aforementioned sensor metrics. For example, if an AIL metric for a particular infusion pump 110 exceeds a safety threshold (e.g., a maximum number of air bubbles within a given amount of time or volume of liquid), the infusion pump 110 may pause a pumping operation corresponding to the AIL detector that measured the AIL metric exceeding the safety threshold. The infusion pump 110 or the control unit 120 may output a notification to notify a clinician that the safety threshold has been exceeded. The particular infusion pump 110 or control unit 120 may output the notification on a display, output the notification as an audio alert from a speaker, and/or transmit the notification to another computing device communicatively coupled to the control unit 120 (e.g., external equipment such as a medical facility server or other computer and with a portable processor, such as a handheld communication device or a laptop-type of computer, or other information device that a clinician may have access to).

Typically, medical fluid administration sets 112 have more parts than are shown in FIG. 1. Many have check valves, drip chambers, valved ports, connectors, and other devices well known to those skilled in the art. These other devices have not been included in the drawings so as to preserve clarity of illustration.

In some implementations, each infusion pump 110 includes a door and a handle that operates to lock the door in a closed position for operation and to unlock and open the door for access to the internal pumping and sensing mechanisms and to load administration sets 112 for the infusion pump. When the door is open, the tube of a respective administration set 112 can be connected with the infusion pump. When the door is closed, the tube is brought into operating engagement with the infusion pumping mechanism, upstream and downstream pressure sensors, and other equipment of the infusion pump. A display, such as an LED display, is located in plain view on the door in this implementation and may be used to visually communicate various information relevant to the infusion pump 110, such as alert indications (e.g., alarm messages).

In some implementations, control keys exist on each infusion pump 110 and the control unit 120 for programming and controlling operations of the infusion pump as desired. In some implementations, the control keys may be presented as interactive elements on a display (e.g., a touchscreen display). The infusion pumps 110 and/or the control unit 120 may also include audio alert equipment in the form of a speaker (not shown).

In some implementations, the control unit 120 includes a display for visually communicating various information, such as the operating parameters of a connected pump and alert indications and alert messages, and control keys for selecting and/or setting control parameters and/or options for controlling the infusion pumps 110 and other connected modules. The control unit 120 may also include a speaker to provide audible alerts. In some implementations, the display may be implemented as a touchscreen display. In such implementations, the control keys may be omitted or reduced in number by providing corresponding interactive elements via a graphical user interface presented via the display. In some implementations, each control key may select a corresponding option displayed in the display.

The control unit 120 may include a communications system (not shown) with which the control unit 120 may communicate with external equipment such as a medical facility server or other computer and with a portable processor, such as a handheld communication device or a laptop-type of computer, or other information device that a clinician may have to transfer information as well as to download drug libraries (e.g., to infusion pumps 110). The communication module may be used to transfer access and interaction information for clinicians encountering the control unit 120 or infusion pumps 110 coupled therewith. The communications system may include one or more of a radio frequency (RF) system, an optical system (e.g., infrared), a Bluetooth system, or other wired or wireless system. The communications system may additionally or alternatively be included integrally with an infusion pump 110, such as in cases where a control unit 120 is not used.

Additionally, other types of modules may be connected to the infusion pumps 110 and/or the control unit 120, such as a syringe pump module, a patient-controlled analgesic module, an external safety device (ESD), a patient safety device such as an end tidal CO2 monitoring module or an oximeter monitoring module, or the like. Some ESDs may include, for example, an air trap, an air detector (e.g., an AIL detector as described herein), a flow detector, a particulate detector, or a spectral analyzer.

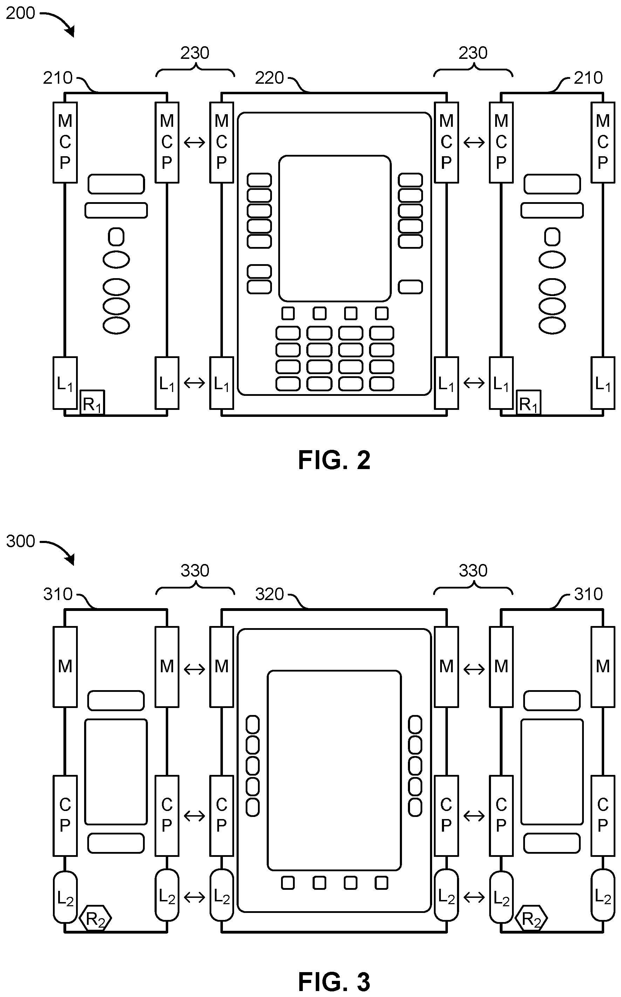

FIG. 2 depicts an infusion pump system 200 having a plurality of subsystems that are joinable using compatible mechanical and electrical interconnects. System 200 includes a plurality of infusion pumps 210 (e.g., corresponding to infusion pumps 110, FIG. 1) and a control unit 220 (e.g., corresponding to control unit 120, FIG. 1). Infusion pumps 210 and control unit 220 in system 200 have the same or similar features as those described above with reference to system 100 (FIG. 1).

The infusion pumps 210, the control unit 220, and any other modules (e.g., discussed above with reference to FIG. 1) may be configured as subsystems of one infusion pump system 200. These subsystems may be coupled to each other using modular interconnect systems 230. The modular interconnect systems 230 may comprise pluralities of connectors affixed to each side of each module, thus allowing the infusion pump system 200 to be modular and configurable for customer needs. The modularity of the connectors may be realized with plug and receptacle connection types (sometimes referred to as male and female connection types), as well as varying degrees of distribution of mechanical (M), communication (C), and power (P) features.

The design and functionality of the connectors in a given interconnect system may depend on the model and version of the modules (infusion pumps 210 and control unit 220) on which they are connected. Thus, various interconnect systems may comprise different types of connectors (also referred to as interconnects), each having different functions. While interconnect systems of different subsystems may be different, the connectors in each system have common functions incorporated into their designs, providing mechanical connections (M), communication connections (C), and power connections (P) between the modules on which they are affixed (e.g., between a control unit 220 and one or more infusion pumps 210). Additional mechanical connections may be made with latches L₁ and corresponding latch releases R₁ (e.g., control components that, when activated, release the latch).

Mechanical connectors M comprise physical connections (e.g., latches) that support physical interoperation between various modules. These connectors form a support structure that supports continuity of attachment for the connected modules upon being connected. As noted above, while the control unit 220 may be mounted to a roller stand or pole (e.g., 130 in FIG. 1), the infusion pumps 210 are mounted to the control unit 220 or to each other. This mounting is enabled by the mechanical connectors M.

Communication connectors C comprise connections that support logical interoperation between various modules. These connectors form a communication bridge that supports the transmitting and receiving of messages between the infusion pumps 210 and the control unit 220. Example messages include infusion instructions sent from the control unit 220 to one or more of the infusion pumps 210 regarding infusion rates, amounts, timing, and so forth. Example messages further include data sent from the infusion pumps 210 to the control unit 220 communicating alarm status, infusion status, and so forth.

Power connectors P comprise connections that support power management between various modules. For implementations in which the infusion pumps 210 depend on the control unit 220 for power, the control unit 220 provides power to the infusion pumps 210 through the power connectors P.

The mechanical, power, and communication connectors MPC may be implemented in system 200 as an inter-unit interface (IUI). Example communication pathways of an IUI include (i) a module detection (MODDET) pathway configured to control power to the connected modules, (ii) transmit and receive (TxD and RxD) pathways configured to handle communication between the control unit and connected modules, (iii) Unit ID Detect pathways configured to allow the control unit to determine when modules are attached, and (iv) Unit ID Enable pathways configured to control access to the system channels.

An IUI connector integrates the mechanical connections M with the power P and communication C connections into a single connector MPC (e.g., a single plug or a single corresponding receptacle). Integrating the mechanical, communication, and power connections into a single connector provides simplicity of operation, making the interconnect system simpler to operate, since only one plug or only one receptable provides all of the functionality required for physical and logical interoperation between the various subsystems of the infusion pump system 200. However, a single connector that integrates connections for all of the aforementioned functions is more complicated and may therefore have a shorter lifespan.

FIG. 3 depicts an infusion pump system 300 having a plurality of subsystems that are joinable using compatible mechanical and electrical interconnects. System 300 includes a plurality of infusion pumps 310 (e.g., corresponding to infusion pumps 210, FIG. 2) and a control unit 320 (e.g., corresponding to control unit 220, FIG. 2). Infusion pumps 310 and control unit 320 in system 300 have the same or similar features as those described above with reference to system 200 (FIG. 2), as well as those described above with reference to system 100 (FIG. 1).

The infusion pumps 310, the control unit 320, and any other modules (e.g., discussed above with reference to FIG. 1) may be configured as subsystems of one infusion pump system 300. These subsystems may be coupled to each other using modular interconnect systems 330. The modular interconnect systems 330 may comprise pluralities of connectors affixed to each side of each module, thus allowing the infusion pump system 300 to be modular and configurable for customer needs. The modularity of the connectors may be realized with plug and receptacle connection types (sometimes referred to as male and female connection types), as well as varying degrees of distribution of mechanical (M), communication (C), and power (P) features.

The design and functionality of the connectors in a given interconnect system may depend on the model and version of the modules (infusion pumps 310 and control unit 320) on which they are connected. Thus, various interconnect systems may comprise different types of connectors (also referred to as interconnects), each having different functions. While interconnect systems of different subsystems may be different, the connectors in each system have common functions incorporated into their designs, providing mechanical connections (M), communication connections (C), and power connections (P) between the modules on which they are affixed (e.g., between a control unit 320 and one or more infusion pumps 310). Additional mechanical connections may be made with latches L₂ and corresponding latch releases R₂ (e.g., control components that, when activated, release the latch).

The mechanical connectors M, and the power and communication connectors PC may be implemented in system 300 as a modular interconnect (MIC), in which the mechanical connector (mechanical modular interconnect, M-MIC) is independent of the communication and power connector (electrical modular interconnect, E-MIC). Keeping the mechanical M and power/communication PC connections separate may provide increased reliability, since each physical connector can be optimized for its specific function without making tradeoffs to the physical layout of the connector. In addition, connectors having fewer functions may be simpler to design and implement, leading to increased lifespans. However, the MIC interconnect system as a whole can be more complicated due to the use of multiple connectors (as opposed to just one IUI), which can increase cost due to having more parts in the bill of materials.

In some implementations, systems 200 and 300 may be different generations of an infusion pump system manufactured by the same company, with system 300 considered to have upgraded features (e.g., efficiency and/or safety features). In some implementations, systems 200 and 300 may be infusion pump systems having similar features but manufactured by different companies. At the very least, the subsystems in system 200 (infusion pumps 210 and control unit 220) are configured for mechanical and electrical coupling to each other, and the subsystems in system 300 (infusion pumps 310 and control unit 320) are configured for mechanical and electrical coupling to each other, but the subsystems in one of the two systems 200/300 are not configured for mechanical and/or electrical coupling to subsystems in another of the two systems 200/300 (e.g., infusion pumps 210 are not compatible with control unit 320, and infusion pumps 310 are not compatible with control unit 220).

While systems 200 and 300 implement, for example, IUI and MIC interconnect systems, implementations of each system may additionally or alternatively implement other interconnect systems not described in this application. As such, the IUI and MIC implementations are nonlimiting examples that illustrate different ways to configure connectors for the various subsystems of an infusion pump system. The concepts involved in the descriptions of these example interconnect systems may be extended to other interconnect systems not included in this disclosure.

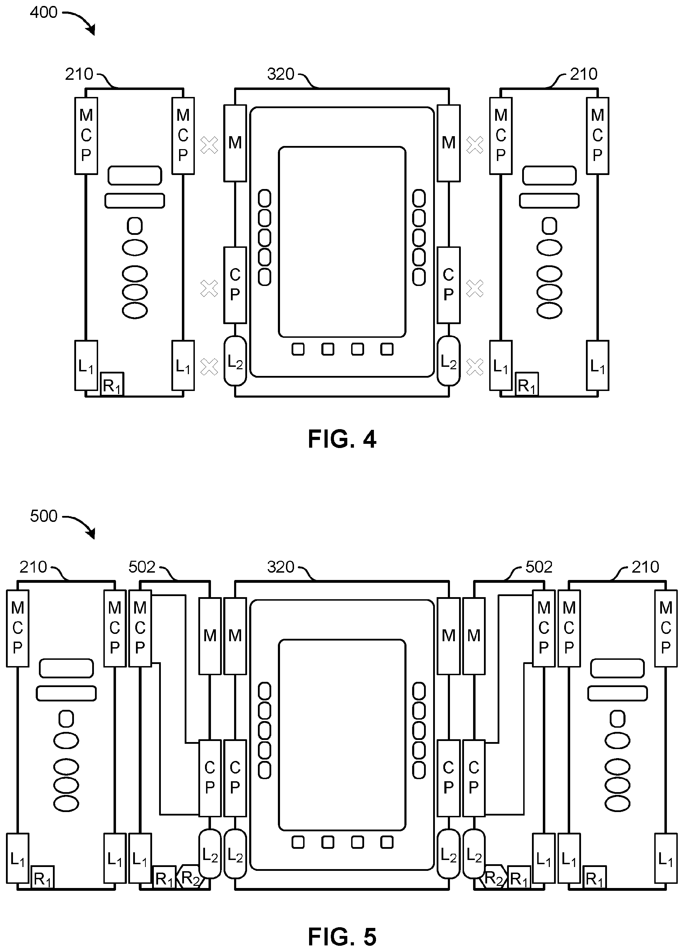

FIG. 4 depicts an infusion pump system 400 having a plurality of subsystems that include connectors that are not joinable. For example, system 400 includes the infusion pumps 210 from system 200 (FIG. 2) and an upgraded control unit 320 from system 300 (FIG. 3). While control unit 320 may provide upgraded functionality and safety features, control unit 320 also includes an interconnect system that is incompatible with the connectors of infusion pumps 210. Specifically, the mechanical connectors M and power/communication connectors PC of the control unit 320 cannot be connected to the mechanical/communication/power MPC connectors of the infusion pumps 210. Moreover, the latches L₂ of the control unit 320 are incompatible with the latches Li and latch releases R₁ of the infusion pumps 210.

System 400 portrays examples illustrating the incompatibility of two different interconnect systems. While system 400 depicts incompatible IUI and MIC connectors, any other pair of incompatible infusion interconnector systems, including those not described in this application, will prevent physical and/or logical interoperation between subsystems of an infusion pump system.

FIG. 5 depicts an infusion pump system 500 having a plurality of subsystems 210/320 having incompatible mechanical and electrical interconnects (as described above with reference to FIG. 4). To make the subsystems compatible with each other, system 500 also includes a plurality of infusion module adapters 502 having sets of interconnects that are compatible with the interconnects of the different subsystems 210/320 of the infusion pump system 500. The adapters 502 provide modularity, physical interoperation, and logical interoperation between the various subsystems 210/320 to which they are physically and communicatively coupled.

System 500 is an example of a mixed-house strategy in which users may migrate from old to new pump subsystems over a series of releases. For example, users may wish to upgrade the control unit (e.g., from control unit 220 in FIG. 2 to control unit 320 in FIG. 3) while carrying their pump modules (e.g., infusion pumps 210 in FIG. 2) forward to the new system, as depicted in system 500. Using older infusion pumps 210 with an upgraded control unit 320 allows users to bridge one product to another (e.g., legacy pumps to new control units), allowing users to have access to the new features of the upgraded control unit without having to replace all of the modules (subsystems) of the entire infusion pump system, thus providing additional functionality at a reduced cost.

The infusion module adapters 502 attach to older modules (e.g., infusion pumps 210) and provide electromechanical interfacing to newer systems (e.g., control unit 320). While system 500 depicts an example in which the adapters 502 couple older infusion pumps 210 to an upgraded control unit 320, other combinations of new and old subsystems are possible. In other implementations, rather than the different subsystems 320/210 representing newer and older modules, they may additionally or alternatively represent modules from different manufacturers, regardless of the generation of technology. Stated another way, the modules/subsystems may be substantially the same generation of technology (equally or substantially equally as old or as new as each other).

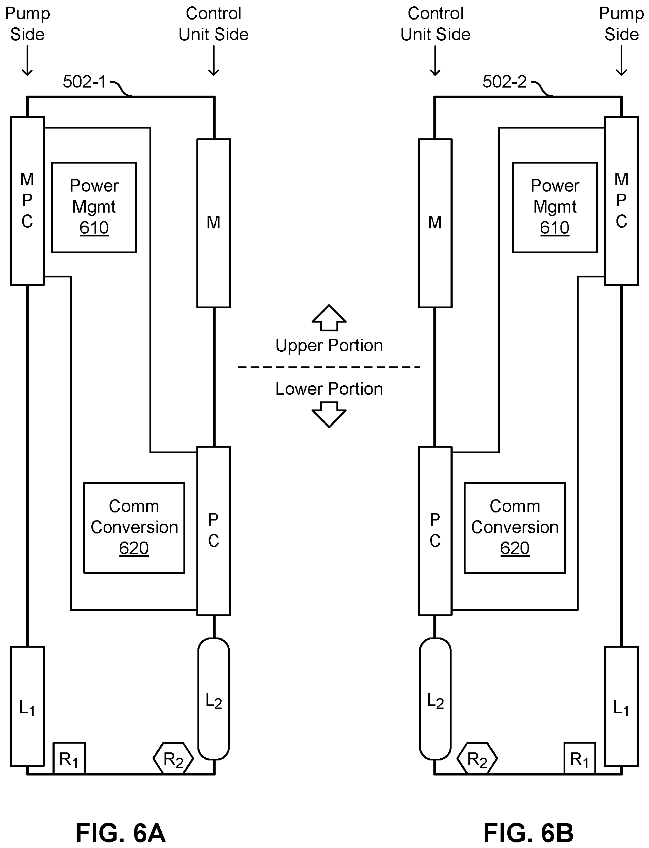

FIGS. 6A-6B depict closeup views of different configurations of an infusion module adapter 502 in accordance with some implementations. In a first configuration (e.g., a "left" configuration) 502-1, the adapter may be configured for coupling to a first of two sides of a control unit (e.g., to the left side of control unit 320 in FIG. 5), and in a second configuration (e.g., a "right" configuration) 502-2, the adapter may be configured for coupling to a second of two sides of a control unit (e.g., to the right side of control unit 320 in FIG. 5).

Both configurations of the adapter 502 include mechanical (physical) connections M, power connections P, and communication (logical) connections C for coupling to corresponding connectors and pathways on the various subsystems to which the adapter is configured to couple.

The mechanical connections M enable physical interoperation between the subsystems that are coupled to the adapter (e.g., control unit 320 and infusion pump 210 in FIG. 5). For example, the mechanical connections M on the control unit side may use a MIC-type connector (in addition to an optional latching mechanism L₂ and corresponding latch release R₂) to physically couple the adapter to the control unit. The mechanical connections on the infusion pump side (M in the MPC connector) may use an IUI-type connector (in addition to an optional latching mechanism L₁ and corresponding latch release R₁) to physically couple the adapter to the infusion pump. In general, a first of two sides of the adapter may use a mechanical connector having a first type (e.g., connector M on the control unit side), and a second of the two sides of the adapter may use a mechanical connector having a second type different from the first type (e.g., connector MPC on the infusion pump side).

The power connections P extend power management between the subsystems that are coupled to the adapter (e.g., control unit 320 and infusion pump 210 in FIG. 5). The adapter includes a power management module 610 that includes electronic circuitry that regulates power levels between the subsystems coupled to each side of the adapter. For example, power circuitry included in the power management module 610 may be configured to regulate power levels between a control area network bus of the control unit 320 and the IUI-type connectors (MPC) on the infusion pump side. Such power circuitry may include voltage step-up converters, voltage step-down converters, and so forth. In addition, the power circuitry included in the power management module 610 may include short-term power storage circuitry (e.g., one or more batteries or capacitors) to allow operation of one subsystem (e.g., infusion pump 210) when not powered by the other subsystem (e.g., control unit 320).

The communication connections C enable logical interoperation between the subsystems (e.g., control unit 320 and infusion pump 210 in FIG. 5) that are coupled to the adapter 502. The adapter includes a communication conversion module 620 that includes electronic circuitry that drives and/or translates communications between the connected subsystems, thereby allowing each subsystem to communicate with the other without the need to change any hardware or software associated with the connected subsystems.

In each configuration 502-1 and 502-2, the adapter includes (i) one or more connectors on a first (control unit) side that are configured for physical and communicative coupling to corresponding connectors on a control unit (e.g., control unit 320 in FIG. 5), and (ii) one or more connectors on a second (pump) side that are configured for physical and communicative coupling to corresponding connectors on a pump module (e.g., infusion pump 210 in FIG. 5).

In some implementations, one of the first and second sides includes only one electromechanical connector (e.g., connector MPC on the infusion pump side), while the other of the first and second sides includes two electromechanical connectors (e.g., connectors M and PC on the control unit side).

In some implementations, one of the first and second sides includes one electromechanical connector in an upper portion of the adapter only or in a lower portion of the adapter only (e.g., connector MPC on the infusion pump side in the upper portion of the adapter), while the other of the first and second sides includes one electromechanical connector in the upper portion of the adapter (e.g., connector M on the control unit side) and one electromechanical connector in a lower portion of the adapter (e.g., connector PC on the control unit side).

In some implementations, one of the first and second sides includes a single electromechanical connector that integrates mechanical connection features (M), power connections (P), and communication pathways (C) (e.g., connector MPC on the infusion pump side), while the other of the first and second sides includes two electromechanical connectors that split the functions of the mechanical, power, and communication features (e.g., connectors M and PC on the control unit side).

In some implementations, the specific connector configuration may vary based on the corresponding connector types and positions on the various subsystems to which the adapter 502 is configured to couple.

In some implementations, the communication conversion module 620 includes message translation circuitry and/or firmware or software programs that translate software messages from one subsystem to be consumable by the other subsystem. For example, module 620 may convert or otherwise process infusion messages from the control unit 320 to be consumable by the infusion pump 210. In another example, module 620 may convert or otherwise process alarms from the infusion pump 210 to be consumable by the control unit 320. This processing of communications allows for data exchange at the physical layer between one subsystem's communication interface (e.g., the control area network bus protocol of the control unit 320) to the other subsystem's communication interface (e.g., to an RS-485 connector of the infusion pump 210). In some implementations, the message translation circuitry may define a workable messaging interface in order to ensure expected messaging behavior between the subsystems (e.g., using different messaging protocols, timing, and so forth).

In some implementations, the communication conversion module 620 implements a state machine that manages state transitions for pump states. In addition, the communication conversion module 620 may include system storage (e.g., 702 as described below) to allow programming updates and facilitate inter-operation for different pumps. For example, the memory may store communication specifications (e.g., timing diagrams and/or logical voltage levels) corresponding to different pump systems, so that the adapter 502 can translate messages between various subsystems in accordance with the stored specifications.

In some implementations, the communication conversion module 620 may include or be communicatively coupled to a wireless radio (e.g., 716 as described below), to allow for updating of firmware, message processing, logging, asset tracking, and so forth.

In some implementations, the adapter 502 may include one or more mechanical switches (e.g., DIP switches) configured to signal to a first subsystem (e.g., control unit) the identity or type of a second subsystem (e.g., pump) to which the adapter is coupled. As such, according to the switch configuration, the control unit may determine the type of the infusion pump that is coupled to the adapter upon detecting presence of the adapter.

In some implementations, the communication conversion module 620 may implement compatibility verification by verifying the cross-compatibility of attached subsystems. This feature may check for authorization (e.g., checking whether a particular control unit is authorized to work with a given pump, or vice versa), software and/or firmware validation (e.g., checking whether the firmware of each subsystem supports the other and/or is updated), and so forth. Specifically, the communication conversion module 620 may determine and confirm software/firmware cross-compatibility, product-level compatibility, and/or version-level compatibility between the control unit 320 and the adapter 502, between the adapter 502 and the infusion pump 210, and/or between the control unit 320 and the infusion pump 210. To that end, the adapter software/firmware may support various versions and generations of control units, pumps, and other modules (e.g., described above with reference to system 100).

In some implementations, the same type of connector (M or C or P) may be disposed on both sides of the adapter 502, while a subset set of the connectors may be implemented as different connector types on either side as described above. For example, the mechanical connector M may be aligned on both sides of the adapter 502 for a universal mechanical connection, but the power and communication connectors P and C may be placed differently or be different connector types for compatibility with different control units and infusion pumps on either side of the adapter 502.

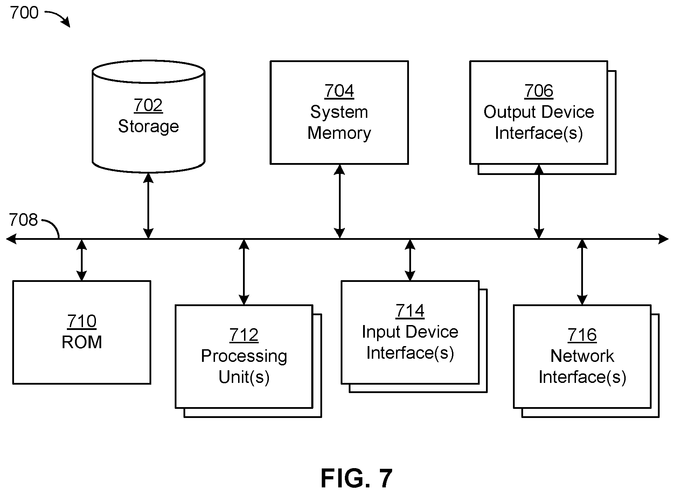

FIG. 7 is a conceptual diagram illustrating an example electronic system 700 for joining subsystems having incompatible interconnects when operating an infusion pump. Electronic system 700 may be a computing device for execution of software, including but not limited to computing hardware within infusion module adapter 502 and/or other specifically configured computing devices or associated terminals disclosed herein. In this regard, electronic system 700 may include the infusion module adapter 502 and/or a computing device within or connected to the infusion module adapter 502.

Electronic system 700 may include various types of computer readable media and interfaces for various other types of computer readable media. In the depicted example, electronic system 700 includes a bus 708, processing unit(s) 712, a system memory 704, a read-only memory (ROM) 710, a permanent storage device 702, input device interface(s) 714, output device interface(s) 706, and network interface(s) 716. In some implementations, electronic system 700 may include or be integrated with other computing devices or circuitry for operation of the various components and methods previously described.

Bus 708 collectively represents all system, peripheral, and chipset buses that communicatively connect the numerous internal devices of electronic system 700. For instance, bus 708 communicatively connects processing unit(s) 712 with ROM 710, system memory 704, and permanent storage device 702.

From these various memory units, processing unit(s) 712 retrieves instructions to execute and data to process, in order to execute the processes of the subject disclosure. The processing unit(s) can be a single processor or a multi-core processor in different implementations.

ROM 710 stores static data and instructions that are needed by processing unit(s) 712 and other modules of the electronic system. Permanent storage device 702, on the other hand, is a read-and-write memory device. This device is a non-volatile memory unit that stores instructions and data even when electronic system 700 is off. Some implementations of the subject disclosure use a mass-storage device (such as a magnetic or optical disk and its corresponding disk drive) as permanent storage device 702.

Other implementations use a removable storage device (such as a floppy disk, flash drive, and its corresponding disk drive) as permanent storage device 702. Like permanent storage device 702, system memory 704 is a read-and-write memory device. However, unlike storage device 702, system memory 704 is a volatile read-and-write memory, such as random-access memory. System memory 704 stores some of the instructions and data that the processor needs at runtime. In some implementations, the processes of the subject disclosure are stored in system memory 704, permanent storage device 702, and/or ROM 710. From these various memory units, processing unit(s) 712 retrieves instructions to execute and data to process, in order to execute the processes of some implementations.

Bus 708 also connects to input and output device interfaces 714 and 706. Input device interface 714 enables the user to communicate information and select commands to the electronic system. Input devices used with input device interface 714 include, e.g., alphanumeric keyboards and pointing devices (also called "cursor control devices"). Output device interfaces 706 enables, e.g., the display of images generated by the electronic system 700. Output devices used with output device interface 706 include, e.g., printers and display devices, such as cathode ray tubes (CRT) or liquid crystal displays (LCD). Some implementations include devices such as a touchscreen that functions as both input and output devices.

Bus 708 also couples electronic system 700 to a network (not shown) through network interfaces 716. Network interfaces 716 may include, e.g., a wireless access point (e.g., Bluetooth or WiFi) or radio circuitry for connecting to a wireless access point. Network interfaces 716 may also include hardware (e.g., Ethernet hardware) for connecting the computer to a part of a network of computers such as a local area network ("LAN"), a wide area network ("WAN"), wireless LAN, or an Intranet, or a network of networks, such as the Internet. Any or all components of electronic system 700 can be used in conjunction with the subject disclosure.

These functions described above can be implemented in computer software, firmware, or hardware. The techniques can be implemented using one or more computer program products. Programmable processors and computers can be included in or packaged as mobile devices. The processes and logic flows can be performed by one or more programmable processors and by one or more programmable logic circuitry. General and special purpose computing devices and storage devices can be interconnected through communication networks.

Some implementations include electronic components, such as microprocessors, storage and memory that store computer program instructions in a machine-readable or computer-readable medium (also referred to as computer-readable storage media, machine-readable media, or machine-readable storage media). Some examples of such computer-readable media include RAM, ROM, read-only compact discs (CD-ROM), recordable compact discs (CD-R), rewritable compact discs (CD-RW), read-only digital versatile discs (e.g., DVD-ROM, dual-layer DVD-ROM), a variety of recordable/rewritable DVDs (e.g., DVD-RAM, DVD-RW, DVD+RW, etc.), flash memory (e.g., SD cards, mini-SD cards, micro-SD cards, etc.), magnetic and/or solid state hard drives, read-only and recordable Blu-Ray^{®} discs, ultra density optical discs, other optical or magnetic media, and floppy disks. The computer-readable media can store a computer program that is executable by at least one processing unit and includes sets of instructions for performing various operations. Examples of computer programs or computer code include machine code, such as is produced by a compiler, and files including higher-level code that are executed by a computer, an electronic component, or a microprocessor using an interpreter.

While the above discussion primarily refers to microprocessor or multi-core processors that execute software, some implementations are performed by one or more integrated circuits, such as application specific integrated circuits (ASICs) or field programmable gate arrays (FPGAs). In some implementations, such integrated circuits execute instructions that are stored on the circuit itself.

As used in this specification and any claims of this application, the terms "computer", "server", "processor", and "memory" all refer to electronic or other technological devices. These terms exclude people or groups of people. For the purposes of the specification, the terms display or displaying means displaying on an electronic device. As used in this specification and any claims of this application, the terms "computer readable medium" and "computer readable media" are entirely restricted to tangible, physical objects that store information in a form that is readable by a computer. These terms exclude any wireless signals, wired download signals, and any other ephemeral signals.

To provide for interaction with a user, implementations of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; e.g., feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user, e.g., by sending web pages to a web browser on a user's client device in response to requests received from the web browser.

Implementations of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), an inter-network (e.g., the Internet), and peer-to-peer networks (e.g., ad hoc peer-to-peer networks).

The computing system can include clients and servers. A client and server are generally remote from each other and may interact through a communication network. The relationship of client and server arises by virtue of computer programs running on the respective computers and having a client-server relationship to each other. In some implementations, a server transmits data (e.g., an HTML page) to a client device (e.g., for purposes of displaying data to and receiving user input from a user interacting with the client device). Data generated at the client device (e.g., a result of the user interaction) can be received from the client device at the server.

Those of skill in the art would appreciate that the various illustrative blocks, modules, elements, components, methods, and algorithms described herein may be implemented as electronic hardware, computer software, or combinations of both. To illustrate this interchangeability of hardware and software, various illustrative blocks, modules, elements, components, methods, and algorithms have been described above generally in terms of their functionality. Whether such functionality is implemented as hardware or software, depends upon the particular application and design constraints imposed on the overall system. The described functionality may be implemented in varying ways for each particular application. Various components and blocks may be arranged differently (e.g., arranged in a different order, or partitioned in a different way) all without departing from the scope of the subject technology.

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order and are not meant to be limited to the specific order or hierarchy presented.

### Further Consideration

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order and are not meant to be limited to the specific order or hierarchy presented.

The previous description is provided to enable any person skilled in the art to practice the various aspects described herein. The previous description provides various examples of the subject technology, and the subject technology is not limited to these examples. Various modifications to these aspects will be readily apparent to those skilled in the art, and the generic principles defined herein may be applied to other aspects. Thus, the claims are not intended to be limited to the aspects shown herein but is to be accorded the full scope consistent with the language claims, wherein reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention described herein.

The predicate words "configured to", "operable to", and "programmed to" do not imply any particular tangible or intangible modification of a subject, but, rather, are intended to be used interchangeably. For example, a processor configured to monitor and control an operation or a component, may also mean the processor being programmed to monitor and control the operation or the processor being operable to monitor and control the operation. Likewise, a processor configured to execute code can be construed as a processor programmed to execute code or operable to execute code.

The term automatic, as used herein, may include performance by a computer or machine without user intervention, for example, by instructions responsive to a predicate action by the computer or machine or other initiation mechanism. The word "example" is used herein to mean "serving as an example or illustration." Any aspect or design described herein as "example" is not necessarily to be construed as preferred or advantageous over other aspects or designs.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "implementation" does not imply that such implementation is essential to the subject technology or that such implementation applies to all configurations of the subject technology. A disclosure relating to an implementation may apply to all implementations, or one or more implementations. An implementation may provide one or more examples. A phrase such as an "implementation" may refer to one or more implementations and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such as a "configuration" may refer to one or more configurations and vice versa.

As used herein a "user interface" (also referred to as an interactive user interface, a graphical user interface or a UI) may refer to a network based interface including data fields and/or other control elements for receiving input signals or providing electronic information and/or for providing information to the user in response to any received input signals. Control elements may include dials, buttons, icons, selectable areas, or other perceivable indicia presented via the UI that, when interacted with (e.g., clicked, touched, selected, etc.), initiates an exchange of data for the device presenting the UI. A UI may be implemented in whole or in part using technologies such as hyper-text mark-up language (HTML), FLASH^{™}, JAVA^{™}, .NET^{™}, C, C++, web services, or rich site summary (RSS). In some implementations, a UI may be included in a stand-alone client (for example, thick client, fat client) configured to communicate (e.g., send or receive data) in accordance with one or more of the aspects described. The communication may be to or from a medical device or server in communication therewith.

As used herein, the terms "determine" or "determining" encompass a wide variety of actions. For example, "determining" may include calculating, computing, processing, deriving, generating, obtaining, looking up (e.g., looking up in a table, a database or another data structure), ascertaining and the like via a hardware element without user intervention. Also, "determining" may include receiving (e.g., receiving information), accessing (e.g., accessing data in a memory) and the like via a hardware element without user intervention. "Determining" may include resolving, selecting, choosing, establishing, and the like via a hardware element without user intervention.

As used herein, the terms "provide" or "providing" encompass a wide variety of actions. For example, "providing" may include storing a value in a location of a storage device for subsequent retrieval, transmitting a value directly to the recipient via at least one wired or wireless communication medium, transmitting or storing a reference to a value, and the like. "Providing" may also include encoding, decoding, encrypting, decrypting, validating, verifying, and the like via a hardware element.

As used herein, the term "message" encompasses a wide variety of formats for communicating (e.g., transmitting or receiving) information. A message may include a machine-readable aggregation of information such as an XML document, fixed field message, comma separated message, JSON, a custom protocol, or the like. A message may, in some implementations, include a signal utilized to transmit one or more representations of the information. While recited in the singular, it will be understood that a message may be composed, transmitted, stored, received, etc. in multiple parts.

As used herein, the term "selectively" or "selective" may encompass a wide variety of actions. For example, a "selective" process may include determining one option from multiple options. A "selective" process may include one or more of: dynamically determined inputs, preconfigured inputs, or user-initiated inputs for making the determination. In some implementations, an n-input switch may be included to provide selective functionality where n is the number of inputs used to make the selection.

As user herein, the terms "correspond" or "corresponding" encompasses a structural, functional, quantitative and/or qualitative correlation or relationship between two or more objects, data sets, information and/or the like, preferably where the correspondence or relationship may be used to translate one or more of the two or more objects, data sets, information and/or the like so to appear to be the same or equal. Correspondence may be assessed using one or more of a threshold, a value range, fuzzy logic, pattern matching, a machine learning assessment model, or combinations thereof. Correspondence may be based on a series of values such as volume of air accumulated over a period of time.

In some implementations, data generated or detected can be forwarded to a "remote" device or location, where "remote," means a location or device other than the location or device at which the program is executed. For example, a remote location could be another location (e.g., office, lab, etc.) in the same city, another location in a different city, another location in a different state, another location in a different country, etc. As such, when one item is indicated as being "remote" from another, what is meant is that the two items can be in the same room but separated, or at least in different rooms or different buildings, and can be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. Examples of communicating media include radio or infra-red transmission channels as well as a network connection to another computer or networked device, and the internet or including email transmissions and information recorded on websites and the like.

## Claims

1. An infusion module adapter system (502) configured to couple a control unit (320) to an infusion pump (210), the infusion module adapter system comprising:
a first interconnect system on a first side of the adapter system and including:
a mechanical connector (M) including first support contacts and configured for mechanical coupling to a corresponding mechanical connector (M) on the control unit; and
a logical connector (PC) including first power contacts and first communication contacts and configured for logical coupling to a corresponding logical connector (PC) on the control unit;
a second interconnect system on a second side of the adapter system and including:
an integrated connector including second support contacts, second power contacts, and second communication contacts (MPC) and configured for mechanical coupling and logical coupling to a corresponding integrated connector (MPC) on the infusion pump, wherein the second power contacts provide a second power signal that is different from a first power signal received at the first power contacts, and the second communication contacts provide a second data signal that is different from a first data signal received at the first communication contacts, wherein the first power signal is **characterized by** a first voltage, a first amplitude, or a first frequency, and the second power signal is **characterized by** a second voltage, a second amplitude, or a second frequency;
a power management module (610) including power conversion circuitry configured to convert the first power signal received from the control unit via the first power contacts of the logical connector of the first interconnect system to the second power signal for transmission to the infusion pump via the second power contacts of the integrated connector of the second interconnect system; and
a communication conversion module (620) including processing circuitry configured to convert messages between the first communication contacts of the logical connector of the first interconnect system and the second communication contacts of the integrated connector of the second interconnect system.

2. The infusion module adapter system of claim 1, wherein:
the first support contacts of the mechanical connector of the first interconnect system comprise a latching mechanism (L2) that supports physical continuity of attachment to the control unit; and
the second support contacts of the integrated connector of the second interconnect system comprise a latching mechanism (L1) that supports physical continuity of attachment to the infusion pump.

3. The infusion module adapter system of claim 1 or claim 2, wherein:
the first communication contacts of the logical connector of the first interconnect system are configured to send messages to and receive messages from the control unit;
the second communication contacts of the integrated connector of the second interconnect system are configured to send messages to and receive messages from the infusion pump; and
the processing circuitry of the communication conversion module is configured to:
translate the messages received from the control unit into a message format that is consumable by the infusion pump and send translated control unit messages to the infusion pump; and
translate the messages received from the infusion pump into a message format that is consumable by the control unit and send translated infusion pump messages to the control unit.

4. The infusion module adapter system of claim 3, wherein the messages received from the control unit include pumping instructions.

5. The infusion module adapter system of claim 3, wherein the messages received from the infusion pump include pumping status or alarm data.

6. The infusion module adapter system of any one of claims 1-5, wherein the processing circuitry of the communication conversion module is configured to manage state transitions for pump states of the infusion pump.

7. The infusion module adapter system of any one of claims 1-6, wherein the infusion module adapter system further includes a wireless transceiver.

8. The infusion module adapter system of claim 7, wherein the processing circuitry of the communication conversion module is configured to receive firmware updates via the wireless transceiver.

9. The infusion module adapter system of claim 7, wherein the processing circuitry of the communication conversion module is configured to transmit logging and/or asset tracking messages via the wireless transceiver.

10. The infusion module adapter system of any one of claims 1-9, wherein the processing circuitry of the communication conversion module is configured to verify compatibility with the infusion pump upon the infusion pump being attached to the infusion module adapter system.

11. The infusion module adapter system of claim 10, wherein the processing circuitry of the communication conversion module is configured to verify compatibility by validating an authorization code stored in a data storage of the infusion module adapter system.

12. The infusion module adapter system of claim 10, wherein the processing circuitry of the communication conversion module is configured to verify compatibility by validating a firmware or software version of (i) the control unit and the infusion module adapter system, (ii) the infusion module adapter system and the infusion pump, and/or (iii) the control unit and the infusion pump.

13. The infusion module adapter system of claim 10, wherein the processing circuitry of the communication conversion module is configured to verify compatibility by validating cross-compatibility, product-level compatibility, and/or version-level compatibility of (i) the control unit and the infusion module adapter system, (ii) the infusion module adapter system and the infusion pump, and/or (iii) the control unit and the infusion pump.

14. The infusion module adapter system of any one of claims 1-13, wherein:
the mechanical connector of the first interconnect system and the integrated connector of the second interconnect system are disposed in an upper portion of the infusion module adapter system; and
the logical connector of the first interconnect system is disposed in a lower portion of the infusion module adapter system.

15. The infusion module adapter system of any one of claims 1-14, wherein:
the mechanical connector of the first interconnect system is disposed in an upper portion of the infusion module adapter system; and
the logical connector of the first interconnect system and the integrated connector of the second interconnect system are disposed in a lower portion of the infusion module adapter system.

## Patentansprüche

1. Infusionsmodul-Adaptersystem (502), das dazu ausgebildet ist, eine Steuereinheit (320) mit einer Infusionspumpe (210) zu koppeln, wobei das Infusionsmodul-Adaptersystem Folgendes umfasst:
ein erstes Verbindungssystem an einer ersten Seite des Adaptersystems, umfassend:
einen mechanischen Konnektor (M), der erste Stützkontakte umfasst und zur mechanischen Kopplung an einen entsprechenden mechanischen Konnektor (M) an der Steuereinheit ausgebildet ist; und
einen logischen Konnektor (PC), der erste Leistungskontakte und erste Kommunikationskontakte umfasst und zur logischen Kopplung an einen entsprechenden logischen Konnektor (PC) an der Steuereinheit ausgebildet ist;
ein zweites Verbindungssystem an einer zweiten Seite des Adaptersystems, umfassend:
einen integrierten Konnektor, der zweite Stützkontakte, zweite Leistungskontakte und zweite Kommunikationskontakte (MPC) umfasst und zur mechanischen Kopplung und zur logischen Kopplung an einen entsprechenden integrierten Konnektor (MPC) an der Infusionspumpe ausgebildet ist, wobei die zweiten Leistungskontakte ein zweites Leistungssignal bereitstellen, das sich von einem ersten Leistungssignal unterscheidet, das an den ersten Leistungskontakten empfangen wird, und die zweiten Kommunikationskontakte ein zweites Datensignal bereitstellen, das sich von einem ersten Datensignal unterscheidet, das an den ersten Kommunikationskontakten empfangen wird, wobei das erste Leistungssignal durch eine erste Spannung, eine erste Amplitude oder eine erste Frequenz gekennzeichnet ist und das zweite Leistungssignal durch eine zweite Spannung, eine zweite Amplitude oder eine zweite Frequenz gekennzeichnet ist;
ein Leistungsmanagementmodul (610), das eine Leistungskonvertierungsschaltung umfasst, die dazu ausgebildet ist, das erste Leistungssignal, das von der Steuereinheit über die ersten Leistungskontakte des logischen Konnektors des ersten Verbindungssystems empfangen wird, in das zweite Leistungssignal zur Übertragung an die Infusionspumpe über die zweiten Leistungskontakte des integrierten Konnektors des zweiten Verbindungssystems umzuwandeln; und
ein Kommunikationsumwandlungsmodul (620), das eine Verarbeitungsschaltung umfasst, die dazu ausgebildet ist, Nachrichten zwischen den ersten Kommunikationskontakten des logischen Konnektors des ersten Verbindungssystems und den zweiten Kommunikationskontakten des integrierten Konnektors des zweiten Verbindungssystems umzuwandeln.

2. Infusionsmodul-Adaptersystem nach Anspruch 1, wobei:
die ersten Stützkontakte des mechanischen Konnektors des ersten Verbindungssystems einen Verriegelungsmechanismus (L2) umfassen, der die fortbestehende physische Befestigung an der Steuereinheit unterstützt; und
die zweiten Stützkontakte des integrierten Konnektors des zweiten Verbindungssystems einen Verriegelungsmechanismus (L1) umfassen, der die fortbestehende physische Befestigung an der Infusionspumpe unterstützt.

3. Infusionsmodul-Adaptersystem nach Anspruch 1 oder 2, wobei:
die ersten Kommunikationskontakte des logischen Konnektors des ersten Verbindungssystems dazu ausgebildet sind, Nachrichten an die Steuereinheit zu senden und Nachrichten von der Steuereinheit zu empfangen;
die zweiten Kommunikationskontakte des integrierten Konnektors des zweiten Verbindungssystems dazu ausgebildet sind, Nachrichten an die Infusionspumpe zu senden und Nachrichten von der Infusionspumpe zu empfangen; und
die Verarbeitungsschaltung des Kommunikationsumwandlungsmoduls dazu ausgebildet ist:
die von der Steuereinheit empfangenen Nachrichten in ein Nachrichtenformat zu übersetzen, das von der Infusionspumpe verarbeitet werden kann, und übersetzte Steuereinheitsnachrichten an die Infusionspumpe zu senden; und
die von der Infusionspumpe empfangenen Nachrichten in ein Nachrichtenformat zu übersetzen, das von der Steuereinheit verarbeitet werden kann, und übersetzte Infusionspumpennachrichten an die Steuereinheit zu senden.

4. Infusionsmodul-Adaptersystem nach Anspruch 3, wobei die von der Steuereinheit empfangenen Nachrichten Pumpanweisungen umfassen.

5. Infusionsmodul-Adaptersystem nach Anspruch 3, wobei die von der Infusionspumpe empfangenen Nachrichten Pumpstatus- oder Alarmdaten umfassen.

6. Infusionsmodul-Adaptersystem nach einem der Ansprüche 1 bis 5, wobei die Verarbeitungsschaltung des Kommunikationsumwandlungsmoduls dazu ausgebildet ist, Zustandsübergänge für Pumpenzustände der Infusionspumpe zu verwalten.

7. Infusionsmodul-Adaptersystem nach einem der Ansprüche 1 bis 6, wobei das Infusionsmodul-Adaptersystem ferner einen drahtlosen Transceiver umfasst.

8. Infusionsmodul-Adaptersystem nach Anspruch 7, wobei die Verarbeitungsschaltung des Kommunikationsumwandlungsmoduls dazu ausgebildet ist, Firmware-Updates über den drahtlosen Transceiver zu empfangen.

9. Infusionsmodul-Adaptersystem nach Anspruch 7, wobei die Verarbeitungsschaltung des Kommunikationsumwandlungsmoduls dazu ausgebildet ist, Protokollierungs- und/oder Asset-Tracking-Nachrichten über den drahtlosen Transceiver zu übertragen.

10. Infusionsmodul-Adaptersystem nach einem der Ansprüche 1 bis 9, wobei die Verarbeitungsschaltung des Kommunikationsumwandlungsmoduls dazu ausgebildet ist, die Kompatibilität mit der Infusionspumpe zu verifizieren, wenn die Infusionspumpe an das Infusionsmodul-Adaptersystem angebracht wird.

11. Infusionsmodul-Adaptersystem nach Anspruch 10, wobei die Verarbeitungsschaltung des Kommunikationsumwandlungsmoduls dazu ausgebildet ist, die Kompatibilität durch Validieren eines Autorisierungscodes zu verifizieren, der in einem Datenspeicher des Infusionsmodul-Adaptersystems gespeichert ist.

12. Infusionsmodul-Adaptersystem nach Anspruch 10, wobei die Verarbeitungsschaltung des Kommunikationsumwandlungsmoduls dazu ausgebildet ist, die Kompatibilität durch Validieren einer Firmware- oder Softwareversion von (i) der Steuereinheit und dem Infusionsmodul-Adaptersystem, (ii) dem Infusionsmodul-Adaptersystem und der Infusionspumpe und/oder (iii) der Steuereinheit und der Infusionspumpe zu verifizieren.

13. Infusionsmodul-Adaptersystem nach Anspruch 10, wobei die Verarbeitungsschaltung des Kommunikationsumwandlungsmoduls dazu ausgebildet ist, die Kompatibilität durch Validieren der Kreuzkompatibilität, der Produktkompatibilität und/oder der Versionskompatibilität von (i) der Steuereinheit und dem Infusionsmodul-Adaptersystem, (ii) dem Infusionsmodul-Adaptersystem und der Infusionspumpe und/oder (iii) der Steuereinheit und der Infusionspumpe zu verifizieren.

14. Infusionsmodul-Adaptersystem nach einem der Ansprüche 1 bis 13, wobei:
der mechanische Konnektor des ersten Verbindungssystems und der integrierte Konnektor des zweiten Verbindungssystems in einem oberen Abschnitt des Infusionsmodul-Adaptersystems angeordnet sind; und
der logische Konnektor des ersten Verbindungssystems in einem unteren Abschnitt des Infusionsmodul-Adaptersystems angeordnet ist.

15. Infusionsmodul-Adaptersystem nach einem der Ansprüche 1 bis 14, wobei:
der mechanische Konnektor des ersten Verbindungssystems in einem oberen Abschnitt des Infusionsmodul-Adaptersystems angeordnet ist; und
der logische Konnektor des ersten Verbindungssystems und der integrierte Konnektor des zweiten Verbindungssystems in einem unteren Abschnitt des Infusionsmodul-Adaptersystems angeordnet sind.

## Revendications

1. Système adaptateur de module de perfusion (502) configuré pour coupler une unité de commande (320) à une pompe de perfusion (210), le système adaptateur de module de perfusion comprenant :
un premier système d'interconnexion disposé sur un premier côté du système adaptateur et comprenant :
un connecteur mécanique (M) comprenant des premiers contacts de support et configuré pour un couplage mécanique à un connecteur mécanique (M) correspondant sur l'unité de commande ; et
un connecteur logique (PC) comprenant des premiers contacts d'alimentation et des premiers contacts de communication et configuré pour un couplage logique à un connecteur logique (PC) correspondant sur l'unité de commande ;
un deuxième système d'interconnexion disposé sur un deuxième côté du système adaptateur et comprenant :
un connecteur intégré comprenant des deuxièmes contacts de support, des deuxièmes contacts d'alimentation et des deuxièmes contacts de communication (MPC), et configuré pour un couplage mécanique et un couplage logique à un connecteur intégré (MPC) correspondant sur la pompe de perfusion, les deuxièmes contacts d'alimentation fournissant un deuxième signal d'alimentation différent d'un premier signal d'alimentation reçu aux premiers contacts d'alimentation, et les deuxièmes contacts de communication fournissant un deuxième signal de données différent d'un premier signal de données reçu aux premiers contacts de communication, le premier signal d'alimentation étant **caractérisé par** une première tension, une première amplitude ou une première fréquence, et le deuxième signal d'alimentation étant **caractérisé par** une deuxième tension, une deuxième amplitude ou une deuxième fréquence ;
un module de gestion de l'alimentation (610) comprenant un circuit de conversion de puissance configuré pour convertir le premier signal d'alimentation, reçu de l'unité de commande via les premiers contacts d'alimentation du connecteur logique du premier système d'interconnexion, en le deuxième signal d'alimentation destiné à être transmis à la pompe de perfusion via les deuxièmes contacts d'alimentation du connecteur intégré du deuxième système d'interconnexion ; et
un module de conversion de communication (620) comprenant un circuit de traitement configuré pour convertir des messages entre les premiers contacts de communication du connecteur logique du premier système d'interconnexion et les deuxièmes contacts de communication du connecteur intégré du deuxième système d'interconnexion.

2. Système adaptateur de module de perfusion selon la revendication 1, dans lequel :
les premiers contacts de support du connecteur mécanique du premier système d'interconnexion comprennent un mécanisme de verrouillage (L2) qui assure la continuité physique de la fixation à l'unité de commande ; et
les deuxièmes contacts de support du connecteur intégré du deuxième système d'interconnexion comprennent un mécanisme de verrouillage (L1) qui assure la continuité physique de la fixation à la pompe de perfusion.

3. Système adaptateur de module de perfusion selon la revendication 1 ou 2, dans lequel :
les premiers contacts de communication du connecteur logique du premier système d'interconnexion sont configurés pour envoyer des messages à l'unité de commande et recevoir des messages de l'unité de commande ;
les deuxièmes contacts de communication du connecteur intégré du deuxième système d'interconnexion sont configurés pour envoyer des messages à la pompe de perfusion et recevoir des messages de la pompe de perfusion ; et
le circuit de traitement du module de conversion de communication est configuré pour :
traduire les messages reçus de l'unité de commande dans un format de message pouvant être traité par la pompe de perfusion et envoyer les messages traduits de l'unité de commande à la pompe de perfusion ; et
traduire les messages reçus de la pompe de perfusion dans un format de message pouvant être traité par l'unité de commande et envoyer les messages traduits de la pompe de perfusion à l'unité de commande.

4. Système adaptateur de module de perfusion selon la revendication 3, dans lequel les messages reçus de l'unité de commande comprennent des instructions de pompage.

5. Système adaptateur de module de perfusion selon la revendication 3, dans lequel les messages reçus de la pompe de perfusion comprennent des données d'état de pompage ou des données d'alarme.

6. Système adaptateur de module de perfusion selon l'une quelconque des revendications 1 à 5, dans lequel le circuit de traitement du module de conversion de communication est configuré pour gérer des transitions d'état pour des états de pompe de la pompe de perfusion.

7. Système adaptateur de module de perfusion selon l'une quelconque des revendications 1 à 6, dans lequel le système adaptateur de module de perfusion comprend en outre un émetteur-récepteur sans fil.

8. Système adaptateur de module de perfusion selon la revendication 7, dans lequel le circuit de traitement du module de conversion de communication est configuré pour recevoir des mises à jour de micrologiciel via l'émetteur-récepteur sans fil.

9. Système adaptateur de module de perfusion selon la revendication 7, dans lequel le circuit de traitement du module de conversion de communication est configuré pour transmettre des messages de journalisation et/ou de suivi d'actifs via l'émetteur-récepteur sans fil.

10. Système adaptateur de module de perfusion selon l'une quelconque des revendications 1 à 9, dans lequel le circuit de traitement du module de conversion de communication est configuré pour vérifier la compatibilité avec la pompe de perfusion lors de la fixation de la pompe de perfusion au système adaptateur de module de perfusion.

11. Système adaptateur de module de perfusion selon la revendication 10, dans lequel le circuit de traitement du module de conversion de communication est configuré pour vérifier la compatibilité en validant un code d'autorisation stocké dans un dispositif de stockage de données du système adaptateur de module de perfusion.

12. Système adaptateur de module de perfusion selon la revendication 10, dans lequel le circuit de traitement du module de conversion de communication est configuré pour vérifier la compatibilité en validant une version de micrologiciel ou de logiciel de (i) l'unité de commande et du système adaptateur de module de perfusion, (ii) du système adaptateur de module de perfusion et de la pompe de perfusion, et/ou (iii) de l'unité de commande et de la pompe de perfusion.

13. Système adaptateur de module de perfusion selon la revendication 10, dans lequel le circuit de traitement du module de conversion de communication est configuré pour vérifier la compatibilité en validant une compatibilité croisée, une compatibilité au niveau produit et/ou une compatibilité au niveau version de (i) l'unité de commande et du système adaptateur de module de perfusion, (ii) du système adaptateur de module de perfusion et de la pompe de perfusion, et/ou (iii) de l'unité de commande et de la pompe de perfusion.

14. Système adaptateur de module de perfusion selon l'une quelconque des revendications 1 à 13, dans lequel :
le connecteur mécanique du premier système d'interconnexion et le connecteur intégré du deuxième système d'interconnexion sont disposés dans une partie supérieure du système adaptateur de module de perfusion ; et
le connecteur logique du premier système d'interconnexion est disposé dans une partie inférieure du système adaptateur de module de perfusion.

15. Système adaptateur de module de perfusion selon l'une quelconque des revendications 1 à 14, dans lequel :
le connecteur mécanique du premier système d'interconnexion est disposé dans une partie supérieure du système adaptateur de module de perfusion ; et
le connecteur logique du premier système d'interconnexion et le connecteur intégré du deuxième système d'interconnexion sont disposés dans une partie inférieure du système adaptateur de module de perfusion.
